Europäisches Patentamt

European Patent Office

Office européen des brevets

(19)

(11) Numéro de publication: **0 235 227**
**B1**

(12) **FASCICULE DE BREVET EUROPEEN**

(45) Date de publication du fascicule du brevet: **23.05.90**

(51) Int. Cl.⁵: **G 01 N 33/80**

(21) Numéro de dépôt: **86905312.4**

(22) Date de dépôt: **03.09.86**

(86) Numéro de dépôt international:
**PCT/FR86/00297**

(87) Numéro de publication internationale:
**WO 87/01461 12.03.87 Gazette 87/06**

(54) **DISPOSITIF POUR LA DETERMINATION D'UN GROUPE SANGUIN.**

(30) Priorité: **04.09.85 FR 8513140**
**27.05.86 BE 6048228**

(43) Date de publication de la demande:
**09.09.87 Bulletin 87/37**

(45) Mention de la délivrance du brevet:
**23.05.90 Bulletin 90/21**

(84) Etats contractants désignés:
**BE CH DE FR GB IT LI LU NL SE**

(56) Documents cités:
**EP-A-0 054 087**
**EP-A-0 104 881**

(73) Titulaire: **LACAILLE, Yves**
**12, avenue Duvelleroy**
**F-94130 Nogent sur Marne (FR)**
(73) Titulaire: **DEBEIR, Joelle**
**19, rue Vignon**
**F-75009 Paris (FR)**

(72) Inventeur: **LACAILLE, Yves**
**12, avenue Duvelleroy**
**F-94130 Nogent sur Marne (FR)**
Inventeur: **DEBEIR, Joelle**
**19, rue Vignon**
**F-75009 Paris (FR)**

(74) Mandataire: **Netter, André et al**
**Cabinet NETTER, 40, rue Vignon**
**F-75009 Paris (FR)**

EP 0 235 227 B1

## Description

L'invention concerne un dispositif pour la détermination d'un groupe sanguin, notamment lors d'une transfusion sanguine.

Lorsqu'on procède à une transfusion sanguine, il est prudent de vérifier, immédiatement avant celle-ci, que le sang qu'on s'apprête à transfuser ne présente pas d'incompatibilité avec le sang du receveur, une telle incompatibilité pouvant provenir par exemple d'une erreur d'étiquetage ou d'une erreur dans la transcription du groupe sanguin du receveur.

En particulier, dans certains pays, dont la France, la réglementation exige que cette vérification soit effectuée en ce qui concerne les groupes A/B/O, une incompatibilité à cet égard entraînant des conséquences très graves dans les minutes qui suivent la transfusion.

La détermination des groupes sanguins se fait habituellement en déposant sur une plaque une goutte du sang à identifier et une goutte de sérum-test, l'aspect du mélange obtenu indiquant le groupe. Une variante de cette méthode consiste à utiliser un sérum-test cristallisé sur un support de carton. On dilue alors ce sérum-test par de l'eau; au moment de l'emploi et on y ajoute le sang à tester.

Ces méthodes impliquent des manipulations malcommodes, qui, de plus, s'effectuent à l'air libre, donc dans des conditions d'asepsie douteuses comportant des risques de contaminations pour le manipulateur.

L'invention vise à simplifier la mise en oeuvre des vérifications nécessaires avant une transfusion sanguine.

Un autre but est depermettre cette mise en oeuvre dans des conditions d'hygiène améliorées.

Un autre but encore est de permettre de conserver facilement le résultat du test fixé à la poche de sang qui a été contrôlée.

L'invention a pour objet un dispositif pour la détermination d'un groupe sanguin, caractérisé en ce qu'il comprend au moins une chambre fermée, des moyens pour introduire dans ladite chambre un échantillon de sang à tester pour l'y mettre en contact avec un sérum-test, et des moyens permettant d'observer la réaction du sérum-test avec le sang.

Des moyens peuvent également être prévus pour introduire le sérum-test dans la chambre.

En variante, le sérum-test peut être présent à l'avance dans la chambre, soit à l'état liquide, soit à l'état sec, par exemple sous forme de cristaux ou en tant qu'imprégnant d'un support poreux.

Selon un mode de réalisation de l'invention, la chambre est fermée de façon étanche aux gaz et est placée sous vide avant l'introduction du sang, et les moyens d'introduction du sang comprennent un organe mobile propre à mettre en relation l'intérieur de la chambre avec un récipient contenant le sang à identifier pour permettre l'aspiration d'un échantillon de celui-ci dans la chambre.

On obtient ainsi à coup sûr le transfert dans la chambre d'une quantité de sang qui est déterminée par le degré de vide, c'est-à-dire par la pression résiduelle régnant dans la chambre au moment où celle-ci entre en relation avec le récipient.

Selon une caractéristique de l'invention, l'organe mobile est une aiguille creuse qui vient perforer une paroi de la chambre. Cette aiguille peut présenter à ses deux extrémités des pointes qui perforent respectivement et successivement la paroi du récipient et celle de la chambre.

Selon un mode particulier de réalisation de l'invention, le dispositif comprend un boîtier en deux pièces déplaçables l'une par rapport à l'autre et chacune par rapport à l'aiguille, la chambre et le récipient étant solidaires respectivement des deux pièces et le mouvement relatif de celles-ci amenant successivement la paroi du récipient et celle de la chambre à être perforées par les pointes de l'aiguille.

Avantageusement, le déplacement relatif des deux pièces du boîtier est un coulissement et l'aiguille est guidée dans des canaux des deux pièces alignés selon la direction du coulissement.

Selon un mode d'exécution de l'invention, la paroi perforable de la chambre est définie par un bouchon qui coopère avec l'une des pièces du boîtier pour constituer la chambre étanche.

Le bouchon peut être enfoncé dans un alvéole de la pièce pour l'obturer de façon étanche.

Dans le cas où deux chambres au moins sont prévues, destinées respectivement à l'identification de deux sangs à l'aide d'un même sérum-test, ces deux chambres peuvent être juxtaposées selon la direction d'un tube souple logé dans un évidement de la seconde pièce et constituant le récipient pour ce sang, le déplacement relatif des deux pièces se faisant transversalement à cette direction.

Dans le cas où le dispositif comprend quatre chambres destinées à l'identification de deux sangs différents à l'aide de deux sérums-tests différents, il est avantageux de loger dans le prolongement l'un de l'autre, dans un évidement allongé, deux tubes contenant respectivement les deux sangs à identifier, les quatre chambres étant alignées selon la direction des tubes.

D'autres caractéristiques et avantages de l'invention ressortiront de la description détaillée donnée ci-après et des dessins annexés, dans lesquels:

la figure 1 est une vue en perspective montrant un dispositif selon l'invention, fermé sur un tube souple contenant du sang, et une seringue pour y injecter un échantillon de sang;

la figure 2 est une vue en coupe longitudinale du dispositif montré à la figure 1;

la figure 3 est une vue en perspective montrant le dispositif isolé et ouvert;

la figure 4 est une vue en perspective d'un second dispositif suivant l'invention, montrant deux parties coulissantes légèrement emboîtées;

la figure 5 est une vue en plan du dispositif de la figure 4, les deux parties coulissantes étant dissociées;

la figure 6 est une coupe transversale correspondant à la figure 5; et

les figures 7 à 10 sont des vues en coupe montrant quatre phases du déplacement de l'aiguille mobile.

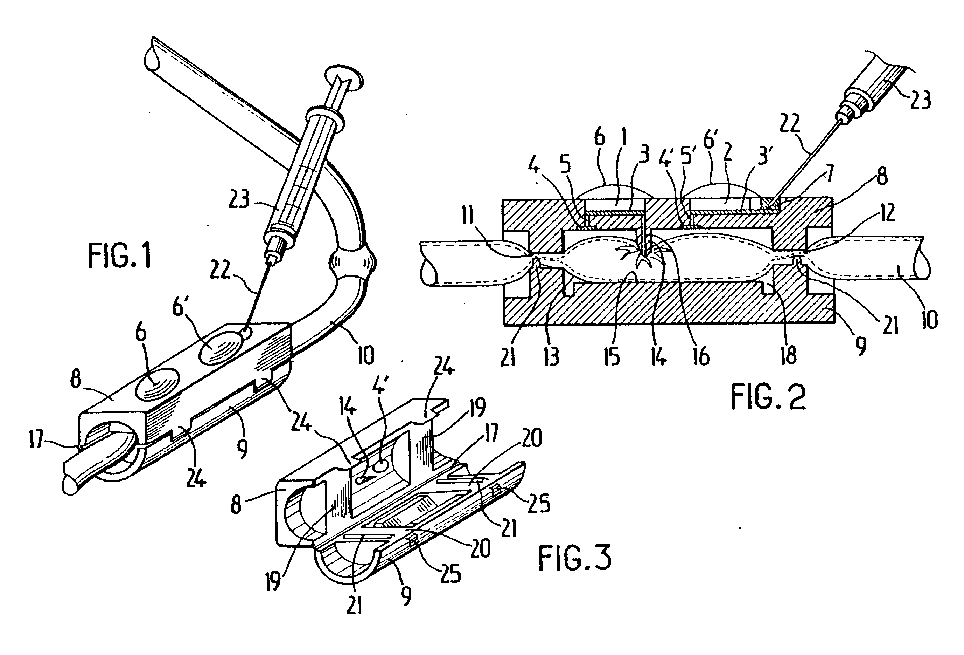

Le dispositif illustré aux figures 1 à 3 comporte deux parties 8 et 9 reliées par une articulation 17 qui leur permet de se rabattre l'une vers l'autre. Lorsqu'elles sont ainsi rabattues, les parties 8 et 9 définissent entre elles un logement 18 pour une portion intermédiaire 13 d'un tuyau souple 10 comprise entre deux zones 11 et 12 pincées par les faces en regard 19 et 20. Des nervures 21 en saillie sur la face 20 de la partie 9 assurent, en coopération avec la face 19 de la partie 8, une fermeture pratiquement étanche de la portion 13 vis-à-vis du reste du tuyau 10.

Une aiguille 14 solidaire de la partie 8 du dispositif fait saillie dans le logement 18. Cette aiguille est percée par une canalisation 16 qui s'étend jusqu'à son extrémité en débouchant dans le logement 18, et relie ce dernier à une chambre fermée 1 prévue dans la partie 8. La chambre 1 est également reliée au logement 18 par une autre canalisation 5 traversée par une membrane hydrophobe 4 perméable à l'air. Dans la chambre 1 est présent un support poreux imprégné d'un sérum-test à l'état sec 3. La chambre 1 est séparée de l'extérieur du dispositif par une loupe 6.

Une autre chambre 2 est ménagée dans la partie 8, à laquelle sont associés un support imprégné de sérum-test sec 3', une canalisation 5', une membrane hydrophobe 4' et une loupe 6' semblables aux éléments décrits sous les numéros de référence 3 à 6 en relation avec la chambre 1. Un élément 7 pouvant être perforé par une aiguille et accessible de l'extérieur du dispositif est adjacent à la chambre 2.

Le fonctionnement du dispositif est le suivant:

L'ouverture du dispositif l'amenant dans la position montrée à la figure 3 permet de mettre en place entre les parties 8 et 9, parallèlement à l'articulation 17, le tuyau souple 10 relié à un réservoir de sang à transfuser et rempli de ce sang. Le tuyau étant mis en place, on rabat sur lui les deux parties 8 et 9 du dispositif comme montré aux figures 1 et 2. La coopération des nervures 21 avec les surfaces 19 isole alors un volume de sang dans la portion intermédiaire 13 du tuyau. Celle-ci s'appuie sur la surface 15 de la partie 9 limitant le logement 18 et faisant face à l'aiguille 14, permettant à celle-ci lors du rabattement de perforer à coup sûr la paroi de la portion 13 du tuyau.

Sous l'effet de la pression régnant dans la portion 13, du sang s'écoule de celle-ci dans la chambre 1, cette pression étant accrue par la diminution de volume due au pincement des zones 11 et 12 du tuyau 10 sur une certaine longueur entre les surfaces 19 et 20 des parties 8 et 9. Le sang remplit l'espace libre de la chambre 1, l'air contenu dans celle-ci s'échappant par la canalisation 5 et à travers la membrane 4, tandis que cette dernière empêche le sang de s'écouler hors de la chambre.

Le sang réagit avec le sérum-test 3 et le résultat peut être observé à travers la loupe 6.

Par ailleurs, on perfore le bouchon 7 au moyen d'une aiguille 22 montée sur une seringue 23 contenant du sang du receveur, et on injecte ainsi ce sang dans la chambre 2. L'évacuation de l'air de la chambre 2 et l'observation de la réaction se font comme décrit à propos de la chambre 1.

Des pattes de verrouillage 24 appartenant à la partie 8, coopérant avec des ergots 25 de la partie 9, maintiennent le dispositif en position de fermeture.

Après avoir constaté la même réaction dans les chambres 1 et 2, on peut procéder à la transfusion. On peut, si on le désire, laisser le dispositif en place sur le tuyau 10, l'aiguille 14 restant plantée dans la paroi de celui-ci. Ceci permet à tout moment, au cours de la transfusion, de vérifier que le test a bien été réalisé et d'en contrôler le résultat.

Le dispositif qui vient d'être décrit permet l'identification du sang au moyen d'un seul sérum-test. Pour la vérification de la compatibilité des sangs à l'égard des groupes A/B/O, qui nécessite la mise en oeuvre de deux sérums-tests anti-A et anti-B, on peut utiliser deux dispositifs, un pour chaque sérum-test.

Une autre solution consiste à utiliser un dispositif modifié comportant quatre chambres, à savoir une paire de chambres semblables à la chambre 1 pour le sang à transfuser et une paire de chambres analogues à la chambre 2 pour le sang du receveur, une chambre de chaque paire contenant le sérum-test anti-A et l'autre le sérum-test anti-B. La disposition relative des quatre chambres dépend du choix de l'homme de métier. Elles peuvent par exemple être placées en une ou deux lignes sous une face unique du dispositif, ou deux à deux sous deux faces adjacentes de celui-ci. Egalement selon le choix de l'homme de métier, les deux chambres destinées au sang à transfuser peuvent être alimentées à partir d'une aiguille unique, par un embranchement de la canalisation, ou de deux aiguilles distinctes.

L'invention englobe également un dispositif comprenant seulement une ou deux chambres destinées au sang à transfuser, utilisables notamment pour tester des unités de sang successives à transfuser à un receveur dont le sang a déjà été testé en même temps que celui de la première unité.

L'invention s'étend également à un dispositif comprenant une, deux ou quatre chambres ou plus munies d'un élément tel que 7 pouvant être perforé par une aiguille, et ne comportant pas d'aiguille incorporée. Un tel dispositif à une ou plusieurs chambres peut être utilisé pour tester un seul sang par un ou plusieurs sérums-tests, par exemple du sang prélevé sur une personne indépendamment de toute opération de transfusion. Ceci permet en particulier de réaliser, avec un nombre de chambres approprié, un test de groupe sanguin complet, tel qu'il est réalisé au laboratoire. Un dispositif comportant un nombre pair de chambres peut être utilisé pour le contrôle de compatibilité avant transfusion, lorsque le réservoir de sang à transfuser n'est pas relié à un tuyau souple pouvant être pincé et perforé, par exemple lorsqu'il s'agit d'un flacon de verre associé à un petit flacon témoin.

Il est possible de prévoir des dispositifs à chambre unique à utiliser en nombre approprié en fonction du test à réaliser, en les mettant en place, le cas échéant, individuellement sur le tuyau souple associé à un réservoir de sang.

Dans tous les cas où un tel tuyau souple n'existe pas, le dispositif peut être simplifié par suppression de l'articulation et de la surface d'appui. Pour l'utilisation en conjonction avec un tuyau souple, les deux parties articulées peuvent être remplacées par deux parties séparées munies de moyens d'assemblage par emboîtement par exemple.

Par ailleurs, l'aiguille 14 perforant le tuyau peut être remplacée par une lame qui l'incise.

Les différentes versions du dispositif selon l'invention peuvent être conservées en emballage stérile jusqu'à leur utilisation, permettant ainsi de réaliser les tests du sang dans des conditions stériles.

Les supports poreux imprégnés de sérum-test 3 peuvent être remplacés par du sérum-test à l'état liquide, ou à l'état de cristaux non supportés. Dans ce dernier cas, la canalisation 16 doit être suffisamment étroite pour retenir les cristaux. Dans le cas du sérum liquide, on peut prévoir un dispositif d'étanchéité formant soupape, à bille par exemple. Lorsque le sérum-test est à l'état sec, il peut être avantageux de prévoir dans la chambre de l'eau pour sa dilution au moment de l'emploi, cette eau pouvant être contenue dans un ou plusieurs récipients brisables qui seront rompus par exemple par enfoncement des lentilles 6 réalisées en matière plastique souple.

Il est également possible de ne pas introduire à l'avance le sérum-test, ou l'eau de dilution, dans les chambres, mais de l'injecter au moment du test à travers un élément tel que 7 associé alors à chacune des chambres, ou de l'introduire en ouvrant les chambres au moyen d'un ou plusieurs couvercles par exemple prévus à cet effet.

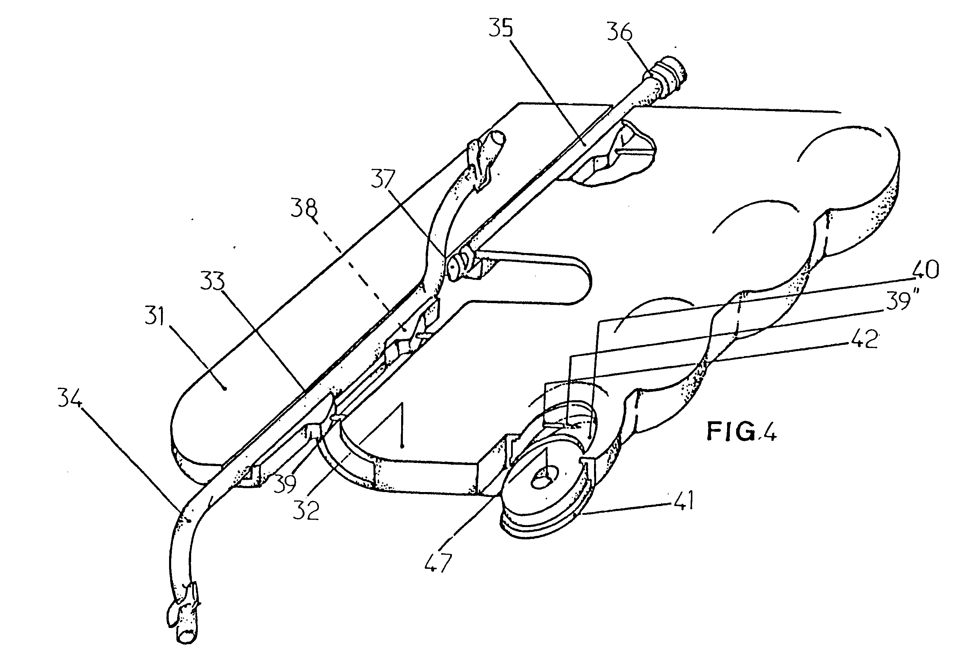

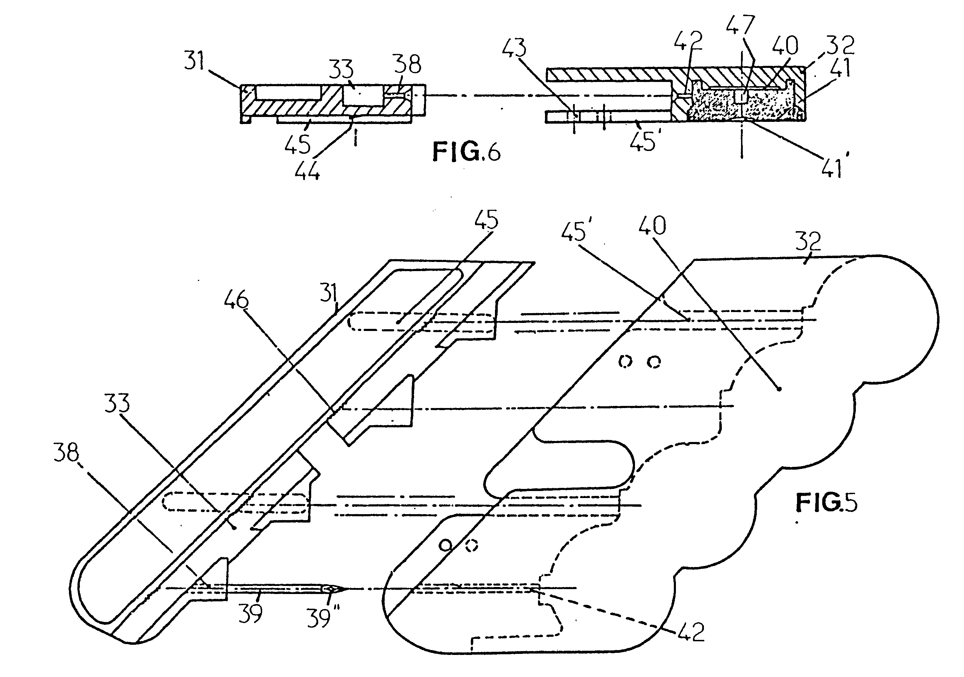

Le dispositif représenté aux figures 4 à 10 comprend deux pièces ou parties de boîtier 31, 32 coulissantes l'une par rapport à l'autre. La partie supérieure 31 présente une rainure 33 destinée à recevoir, d'une part, le tube 34 de la poche à sang (non représentée) et, d'autre part, le tube-témoin 35 destiné à recevoir l'échantillon de sang du receveur.

Le tube-témoin 35 comporte un bouchon 36 perforable par une aiguille pour l'introduction de l'échantillon de sang du receveur et un bouchon perméable 37 pour en évacuer l'air. La partie supérieure comporte en outre des canaux 38 destinés à guider les aiguilles mobiles 39.

La partie inférieure 32 comporte un ensemble de chambres 40 fermées chacune à l'aide d'un bouchon 41 et pourvues chacune d'un canal 42, dans lequel coulisse l'aiguille mobile d'introduction du sang correspondante 39.

Les aiguilles mobiles creuses 39, positionnées à un moment de la fabrication dans les canaux 42 et 38 des parties inférieure 32 et supérieure 31, comportent à chacune de leurs extrémités un biseau orienté 39', respectivement 39''.

Suivant la réalisation représentée, le dispositif comprend un ensemble de chambres séparées l'une de l'autre et, de préférence, des paires de chambres, de manière à tester simultanément le sang conservé et le sang du receveur.

Le dispositif comportera avantageusement quatre chambres, dont une paire pour le sang du donneur et une paire pour le sang du receveur.

Les axes de la rainure 33 de la partie supérieure 31 et des canaux de guidage 38, 42 des aiguilles 39 se croisent suivant un angle, qui peut varier de 30° à 60°. Cette inclinaison des canaux de guidage des aiguilles doit permettre à celles-ci de pénétrer d'abord les tubes-témoins 34, 35 sous un angle favorable réduisant l'effort de pénétration et favorisant en outre l'étanchéité à l'endroit de pénétration.

Le fonctionnement du dispositif décrit est le suivant:

On s'assure au préalable que les chambres 40 sont toujours sous dépression par l'examen du bouchon 41 qui doit montrer un léger affaissement circulaire en son centre 41', la dépression dans les chambres étant obtenue par tout moyen connu et, par exemple, en plaçant le dispositif dans une enceinte sous vide à un moment donné de la fabrication.

L'échantillon de sang du receveur est injecté dans le tube-témoin 35 prévu à cet effet.

Le tube-témoin 34 de la poche à sang est introduit dans la rainure 33 prévue à cet effet et la partie supérieure 31 est enfoncée jusqu'à un premier arrêt 43 où elle est verrouillée par son cran 44.

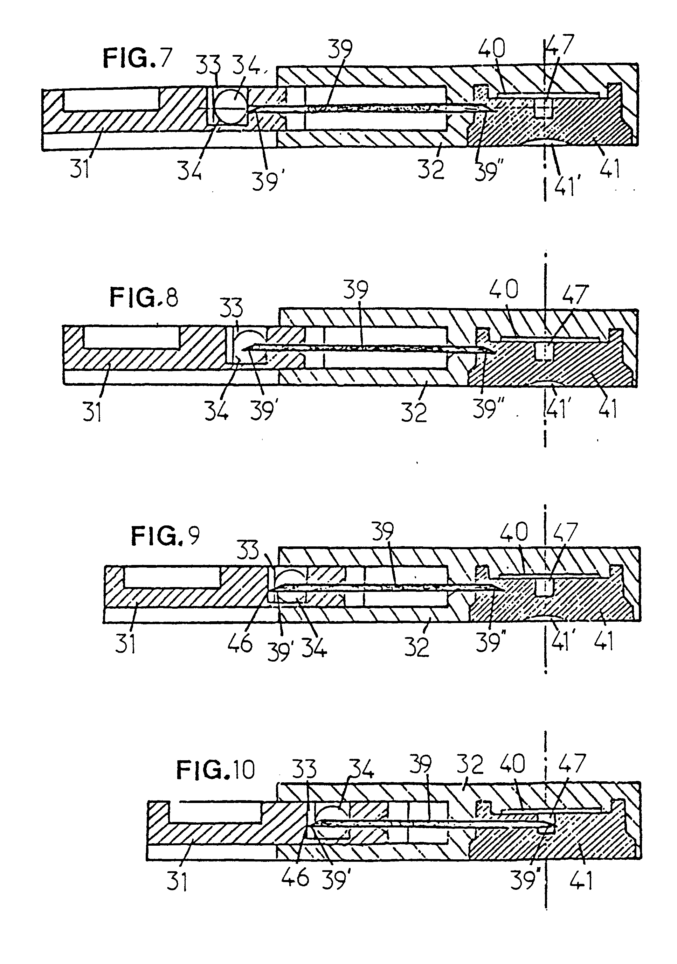

Par une légère pression on fait coulisser la partie supérieure 31 dans la partie inférieure 32 jusqu'à butée, les deux pièces étant guidées l'une par rapport à l'autre au moyen de glissières 45, 45' (figure 8).

Les aiguilles mobiles 9 ont la particularité de perforer en premier lieu les tubes-témoins 34, 35 contenant le sang à tester et de les traverser jusqu'à ce que la pointe du biseau 39' vienne buter contre le fond de la rainure 33. L'angle du biseau et son orientation sont tels que la pointe de l'aiguille 39 va s'accrocher dans une denture 46 prévue à cet effet dans la partie supérieure 31 sans qu'il y ait communication entre le sang contenu dans le tube et l'extérieur du dispositif (figure 9).

En second lieu, sous l'effet du déplacement relatif des deux parties 31 et 32 entre elles, l'aiguille 39 traverse l'épaisseur de la paroi du bouchon 41 jusqu'à déboucher dans la cavité 47 y aménagée et où règne la dépression. Sous l'effet de cette dépression, un volume calibré de sang va être aspiré dans la chambre 40 pour s'y mélanger intimement avec le sérum y préalablement déposé (figure 10).

L'inspection de l'échantillon se fait par examen visuel au travers de la paroi supérieure de la chambre, le dispositif étant réalisé en une matière transparente.

Lors de la fabrication, le sérum-test est introduit dans la ou les chambres 40, de préférence à l'état liquide. Le sérum-test étant introduit dans la chambre, celle-ci est alors mise sous dépression et fermée par le bouchon 41.

La configuration d'une chambre est telle que le mélange sérum-sang se fait de façon homogène et en couche mince et régulière, comme le laisse prévoir la coupe de la chambre en figure 7. Cela facilite l'agglutination et rend la comparaison visuelle entre les chambres plus aisée.

La dépression régnant à l'intérieur de la chambre est réglée de façon à aspirer un volume de sang constant et adapté avec précision au volume de sérum déposé dans la chambre, afin d'obtenir un mélange idéal pour l'agglutination et l'interprétation.

Grâce aux crans 44 et aux arrêts 43, le dispositif est pourvu d'un système de verrouillage des deux parties, afin qu'elles ne puissent être désolidarisées après utilisation. Les deux parties 31, 32 étant verrouillées, le dispositif reste fixé au tube-témoin 34 de la poche à sang, éliminant ainsi toute confusion.

A aucun moment donné des différentes phases les tubesté-moins 34, 35 ne sont soumis à pression et il est donc exclu que du sang suinte vers l'extérieur du dispositif.

Tout comme le dispositif des figures 1 à 3, celui des figures 4 à 10 peut comporter un nombre variable de chambres selon sa destination. De la même façon, chaque chambre peut ne pas contenir de sérum-test ou contenir du sérum test liquide ou sec.

**Revendications**

1. Dispositif pour la détermination d'un groupe sanguin, caractérisé en ce qu'il comprend au moins une chambre fermée (1, 2), des moyens (7, 14, 15, 16) pour introduire dans ladite chambre un échantillon de sang à tester pour l'y mettre en contact avec un sérum-test (3), et des moyens (6) permettant d'observer la réaction du sérum-test avec le sang.

2. Dispositif selon la revendication 1, caractérisé en ce qu'il comprend en outre des moyens pour introduire le sérum-test dans la chambre.

3. Dispositif selon la revendication 1, caractérisé en ce que le sérum-test est présent à l'avance dans la chambre.

4. Dispositif selon la revendication 3, caractérisé en ce que le sérum-test est présent à l'état liquide.

5. Dispositif selon la revendication 3, caractérisé en ce que le sérum-test est présent à l'état sec, par exemple sous forme de cristaux ou en tant qu'imprégnant d'un support poreux.

6. Dispositif selon l'une des revendications précédentes, caractérisé en ce qu'il comprend au moins une paire de chambres, destinées respectivement à l'identification d'un même sang à l'aide de deux sérums-tests différents.

7. Dispositif selon l'une des revendications précédentes, caractérisé en ce qu'il comprend au moins une paire de chambres, destinées respectivement à l'identification, à l'aide d'un même sérum-test, du sang d'un patient devant recevoir une transfusion et du sang destiné à cette transfusion.

8. Dispositif selon la revendication 7, caractérisé en ce qu'il comprend deux paires de chambres destinées respectivement à l'identification à l'aide de deux sérums-tests différents.

9. Dispositif selon l'une des revendications précédentes, caractérisé en ce que la chambre (40) est fermée de façon étanche aux gaz et est placée sous vide avant l'introduction du sang, et que les moyens d'introduction du sang comprennent un organe mobile (39) propre à mettre en relation l'intérieur de la chambre (40) avec un récipient (34) contenant le sang à identifier pour permettre l'aspiration d'un échantillon de celui-ci dans la chambre.

10. Dispositif selon la revendication 9, caractérisé en ce que l'organe mobile est une aiguille creuse qui vient perforer une paroi (41) de la chambre.

11. Dispositif selon la revendication 10, caractérisé en ce que l'aiguille creuse présente des pointes à ses deux extrémités qui perforent respectivement la paroi du récipient (34) et celle de la chambre.

12. Dispositif selon la revendication 11, caractérisé en ce qu'il comprend un boîtier en deux pièces (31, 32) déplaçables l'une par rapport à l'autre et chacune par rapport à l'aiguille, le récipient (34) et la chambre (40) étant solidaires respectivement des deux pièces (31, 32) et le mouvement relatif de celles-ci amenant successivement la paroi du récipient et celle de la chambre à être perforées par les pointes de l'aiguille.

13. Dispositif selon la revendication 12, caractérisé en ce que le déplacement relatif des deux pièces du boîtier est un coulissement et que l'aiguille est guidée dans des canaux (38, 42) des deux pièces alignés selon la direction du coulissement.

14. Dispositif selon l'une des revendications 10 à 13, caractérisé en ce que la paroi perforable de la chambre est définie par un bouchon (41) qui coopère avec la seconde pièce (32) du boîtier pour constituer la chambre étanche.

15. Dispositif selon la revendication 14, caractérisé en ce que la coupelle est enfoncée dans un alvéole de la seconde pièce qu'elle obture de façon étanche.

16. Dispositif selon l'une des revendications 12 à 15, caractérisé en ce que le récipient est un tube souple logé dans un évidement (33) de la seconde pièce.

17. Dispositif selon la revendication 6 et selon la revendication 16, caractérisé en ce que les deux chambres sont juxtaposées selon la direction du tube, le déplacement relatif des deux pièces se faisant transversalement à cette direction.

18. Dispositif selon la revendication 8 et selon la revendication 17, caractérisé en ce que la première pièce présente un évidement allongé (33) propre à recevoir, dans le prolongement l'un de l'autre, deux tubes (34, 35) contenant respectivement les deux sangs à identifier, les quatre chambres étant alignées selon la direction des tubes.

**Patentansprüche**

1. Vorrichtung zur Blutgruppenbestimmung, gekennzeichnet durch mindestens eine geschlossene Kammer (1, 2), Mittel (7, 14, 15, 16) zum Einführen einer Blutprobe in diese Kammer, um sie dort mit einem Testserum (3) in Kontakt zu bringen, sowie Mittel (6), die eine Beobachtung der Reaktion des Testserums mit dem Blut ermöglichen.

2. Vorrichtung nach Anspruch 1, ferner gekennzeichnet durch Mittel zum Einführen des Testserums in die Kammer.

3. Vorrichtung nach Anspruch 1, dadurch gekennzeichnet, daß das Testserum im voraus in der Kammer vorliegt.

4. Vorrichtung nach Anspruch 3, dadurch gekennzeichnet, daß das Testserum in flüssigem Zustand vorliegt.

5. Vorrichtung nach Anspruch 3, dadurch gekennzeichnet, daß das Testserum in trockenem Zustand, beispielsweise in Kristallform oder als Imprägnation eines porösen Trägers vorliegt.

6. Vorrichtung nach einem der vorhergehenden Ansprüche, gekennzeichnet durch mindestens ein Paar zur Identifizierung jeweils einer Probe gleichen Blutes mit Hilfe zweier unterschiedlicher Testseren bestimmter Kammern.

7. Vorrichtung nach einem der vorhergehenden Ansprüche, gekennzeichnet durch mindestens ein Paar Kammern, welche jeweils dazu bestimmt sind, mit Hilfe desselben Testserums das Blut eines Patienten, der eine Transfusion erhalten muß und das für diese Transfusion bestimmte Blut zu identifizieren.

8. Vorrichtung nach Anspruch 7, gekennzeichnet durch zwei Paare von Kammern, welche jeweils zur Identifizierung mit Hilfe zweier unterschiedlicher Testseren bestimmt sind.

9. Vorrichtung nach einem der vorhergehenden Ansprüche, dadurch gekennzeichnet, daß die Kammer (40) gasdicht verschlossen ist und vor der Einführung des Blutes unter Unterdruck steht, und daß die Mittel zum Einführen des Blutes ein bewegliches Organ (39) aufweisen, welches geeignet ist, das Innere der Kammer (40) mit einem das zu identifizierende Blut enthaltenden Gefäß (34) zu verbinden, um das Ansaugen einer Probe desselben in die Kammer zu ermöglichen.

10. Vorrichtung nach Anspruch 9, dadurch gekennzeichnet, daß das bewegliche Organ als Hohlnadel ausgebildet ist, welche eine Wand (41) der Kammer perforiert.

11. Vorrichtung nach Anspruch 10, dadurch gekennzeichnet, daß die Hohlnadel an ihren beiden Enden Spitzen aufweist, welche jeweils die Wand des Gefäßes (34) und die der Kammer perforieren.

12. Vorrichtung nach Anspruch 11, gekennzeichnet durch ein Gehäuse aus zwei Teilen (31, 32), von denen jeweils das eine gegenüber dem anderen und jedes gegenüber der Nadel verschiebbar ist, wobei das Gefäß (34) und die Kammer (40) bezüglich der beiden Teile (31, 32) fest angeordnet sind, und wobei deren Relativbewegung dazu führt, daß nacheinander die Wand des Gefäßes und die der Kammer von den Spitzen der Nadel perforiert werden.

13. Vorrichtung nach Anspruch 12, dadurch gekennzeichnet, daß die Relativverschiebung der beiden Teile des Gehäuses eine Gleitbewegung ist und daß die Nadel in in Richtung der Gleitbewegung fluchtenden Kanälen (38, 42) der beiden Teile geführt ist.

14. Vorrichtung nach einem der Ansprüche 10 bis 13, dadurch gekennzeichnet, daß die perforierbare Wand der Kammer durch einen Stopfen (41) gebildet ist, der unter Bildung der gasdichten Kammer mit dem zweiten Teil (32) des Gehäuses zusammenwirkt.

15. Vorrichtung nach Anspruch 14, dadurch gekennzeichnet, daß der Probetiegel in eine Ausnehmung des zweiten Teils eingepreßt ist, welche er dicht verschließt.

16. Vorrichtung nach einem der Ansprüche 12 bis 15, dadurch gekennzeichnet, daß das Gefäß als biegsamer, in einer Aussparung (33) des zweiten Teils angeordneter Schlauch ausgebildet ist.

17. Vorrichtung nach Anspruch 6 und Anspruch 16, dadurch gekennzeichnet, daß die beiden Kammern in Längsrichtung des Schlauchs hintereinander angeordnet sind, wobei die Relativverschiebung der beiden Teile quer zu dieser Längsrichtung erfolgt.

18. Vorrichtung nach Anspruch 8 und Anspruch 17, dadurch gekennzeichnet, daß das erste Teil eine langgestreckte Aussparung (33) zur Aufnahme zweier Schläuche (34, 35) aufweist, von denen jeweils einer in Verlängerung des anderen angeordnet ist und die jeweils die beiden zu bestimmenden Blutproben enthalten, wobei die vier Kammern in Längsrichtung der Schläuche miteinander fluchten.

**Claims**

1. A blood grouping apparatus, characterised in that it comprises at least one closed chamber (1, 2), means (7, 14, 16) for introducing a blood sample to be tested into the chamber for contact therein with a control serum (3), and means (6) for observing the reaction of the control serum with the blood.

2. An apparatus according to claim 1, characterised in that it also comprises means for introducing the control serum into the chamber.

3. An apparatus according to claim 1, characterised in that the control serum is present beforehand in the chamber.

4. An apparatus according to claim 3, characterised in that the control serum is present in the liquid state.

5. An apparatus according to claim 3, characterised in that the control serum is present in the dry state, for example, in the form of crystals or as an impregnant of a porous support.

6. An apparatus according to any of the previous claims, characterised in that it comprises at least one pair of chambers for identifying a single blood by means of two different control serums.

7. An apparatus according to any of the previous claims, characterised in that it comprises at least one pair of chambers for identifying by means of a single control serum the blood of a patient requiring a transfusion and the blood to be used for the transfusion.

8. An apparatus according to claim 7, characterised in that it comprises two pairs of chambers for identification by means of two different control serums.

9. An apparatus according to any of the previous claims, characterised in that the chamber (40) is closed in gas-tight manner and is exhausted before introduction of the blood and the means for introducing the same comprise a moving member (39) adapted to place the inside of the chamber (40) in communication with a receptacle (34) containing the blood to be identified so that a sample of such blood can be aspirated into the chamber.

10. An apparatus according to claim 9, characterised in that the moving member is a hollow needle which perforates a wall (41) of the chamber.

11. An apparatus according to claim 10, characterised in that the hollow needle has tips at both its ends, one tip piercing the wall of the receptacle (34) and the other tip piercing the wall of the chamber.

12. An apparatus according to claim 11, characterised in that it comprises a casing in two parts (31, 32) which can be moved relatively to one another and each of which can be moved relatively to the needle, the receptacle (34) and the chamber (40) being rigidly secured to the two parts (31, 32 respectively), the relative movement of the two parts leading to the receptacle wall and the chamber wall being pierced consecutively by the needle tips.

13. An apparatus according to claim 12, characterised in that the relative movement of the two parts of the casing is a sliding movement and the needle is guided in channels (38, 42) in the two parts, the channels being aligned in the sliding direction.

14. An apparatus according to any of claims 10-13, characterised in that the pierceable chamber wall is defined by a plug (41) which co-operates with the second casing part (32) to form the hermetic chamber.

15. An apparatus according to claim 14, characterised in that the plug is engaged in a socket in the second part and closes the same hermetically.

16. An apparatus according to any of claims 12-15, characterised in that the receptacle is a flexible tube received in a recess (33) in the second part.

17. An apparatus according to claims 6 and 16, characterised in that the two chambers are juxtaposed in the direction of the tube, the relative movement of the two parts being effective transversely to the latter direction.

18. An apparatus according to claims 8 and 17, characterised in that the first part is formed with an elongate recess (33) adapted to receive in prolongation of one another two tubes (34, 35) containing the respective two bloods to be identified, the four chambers being aligned in the direction of the tubes.

FIG.1

FIG.2

FIG.3

EP 0 235 227 B1

FIG.4

FIG.6

FIG.5

**FIG.7**

**FIG.8**

**FIG.9**

**FIG.10**